# EUROPEAN PATENT APPLICATION

(11) **EP 0 751 119 A1**
(43) Date of publication of application: **02.01.1997**
(21) Application number: 96110180.5
(22) Date of filing: 24.06.1996
(51) Int. Cl.: C07C 269/00, C07C 271/62, C07C 273/18, C07C 275/50, C07C 275/54

(54) **A process for preparing acryloyl carbamates or ureas**

(30) Priority: 26.06.1995 DE 19523169
(71) Applicant: DAINIPPON INK AND CHEMICALS, INC., Itabashi-ku Tokyo (JP)
(72) Inventor: Gaudl, Kai-Uwe, 13467 Berlin (DE); Lachowicz, Artur, 13467 Berlin (DE); Grahe, Gerwald F., 14195 Berlin (DE)
(74) Representative: Kraus, Walter, Dr.

(57) **Abstract**

A process for preparing acryloyl carbamates or ureas by reacting β-haloalkanoyl chlorides with carbamate or urea compounds is described. Reaction takes place in the presence of specific basic compounds which function as traps for the hydrogen halides produced during reaction. By means of the process according to the invention, the disadvantages of the prior art, especially with regard to safety and protection of the environment, are reduced. The compounds which can be prepared according to the invention are suitable as monomers for preparing addition copolymers.

## Description

The invention relates to a process for preparing an acryloyl carbamate or acryloyl urea of the general formula (I) 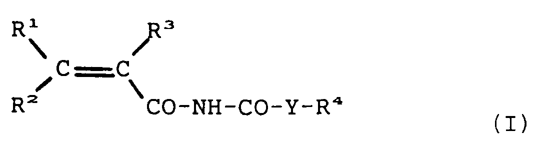 in which R¹, R² and R³ may be identical or different and each represents a hydrogen atom or a methyl or ethyl group, R⁴ represents a straight-chain or branched, saturated or unsaturated aliphatic, cycloaliphatic, arylaliphatic or aromatic hydrocarbon group with 1 to 10 carbon atoms and Y represents an oxygen atom or the NR⁵ group, wherein R⁵ represents a hydrogen atom or a straight-chain or branched, saturated or unsaturated aliphatic, cycloaliphatic, arylaliphatic or aromatic hydrocarbon group with 1 to 6 carbon atoms.

It is known that methacryloyl carbamates and methacryloyl ureas can be prepared from methacryloyl isocyanates and alcohols or amines [EP 0 177 122, Chem. Ber. 84 (1951) 4]. When preparing the corresponding acryloyl carbamates and ureas, however, this process often fails or leads to low yields because the acryloyl isocyanates being used as starting compounds are unstable [Urano et al., Polym. Mater. Sci. Eng., 57 (1987) 583-7]. Acryloyl isocyanate and methacryloyl isocyanate, furthermore, have low molecular weights and are thus highly volatile isocyanates, comparable with volatile and highly toxic methyl, ethyl, propyl and butyl isocyanate. This places especially cost-intensive requirements on the occupational safety measures to be used during reaction. Another economic disadvantage is the comparatively high price of the acryloyl isocyanates used as starting compounds, which is attributed to the fact that acryloyl isocyanates are prepared from relatively expensive oxalyl chloride. The other process for synthesising acryloyl isocyanate by phosgenation of acrylamide (JP 90058257, JP 05032607) involves the known disadvantage of dealing with phosgene.

Further processes for preparing acryloyl carbamates are the reaction of acrylamides with chloroformates and the reaction of acryloyl chlorides with carbamates (JP 04066563). In both processes, strong metallic bases, such as e.g. lithium, sodium, potassium, are used in molar amounts. This is disadvantageous from a technological and economic point of view because these metallic bases require complicated safety precautions due to their capacity for spontaneous ignition and the costs make the syntheses uneconomic as compared with other processes due to the high price of the alkali metals. It is known that the basicity of weaker bases, such as sodium carbonate, is not high enough to activate a corresponding reaction.

A further known method for preparing acryloyl carbamates from acryloyl chlorides and sodium azide in the presence of alcohols (JP 74014547) has the disadvantage that toxic sodium azide forms an unstable intermediate with acid chlorides which can decompose spontaneously and uncontrollably. Therefore this process is not suitable for the synthesis of large amounts. The preparation of acryloyl ureas by transesterification of acryloyl carbamates with amines (JP 93057978) has the disadvantage that the acryloyl carbamates being used have to be prepared first, using known synthetic routes which have the disadvantages described above.

The invention is based on the object of providing a process for preparing acryloyl carbamates and ureas which does not possess the process disadvantages described above and which enables in particular the reliable and environmentally friendly production of the compounds mentioned.

The object of the invention is therefore a process of the type mentioned at the beginning in which a β-haloalkanoyl chloride of the general formula (II) 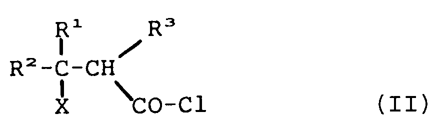 in which R¹, R² and R³ are defined as above and X represents a chlorine or bromine atom
is reacted with a carbamate or urea compound of the general formula (III)

H₂N-CO-Y-R⁴ (III)

in which R⁴ and Y are defined as above, at temperatures of 0 to 100°C and in the presence of a trap for the hydrogen halide produced during reaction.

In accordance with a preferred embodiment of the present invention, the trap for the hydrogen halide produced during reaction is a tertiary amine or an alkali or alkaline earth metal oxide, hydroxide, carbonate, alkoxide and/or carboxylate.

A further embodiment of the invention is characterised in that the trap for the hydrogen halide produced during reaction is added dropwise after initiating the reaction.

Examples of acryloyl carbamates and acryloyl ureas which can be prepared by the process according to the invention are N-acryloylmethylcarbamate, N-acryloylethylcarbamate, N-acryloylpropylcarbamate, N-acryloylbutylcarbamate, N-acryloylphenylcarbamate, N'-acryloyl-N-methylurea, N-acryloylbenzylcarbamate, N'-acryloyl-N -phenylurea and N-methacryloylmethylcarbamate.

Examples of β-haloalkanoyl chlorides of the formula (II) used according to the invention are β-chloropropionyl chloride, β-chloroisobutyryl chloride and β-bromopropionyl chloride, wherein β-chloropropionyl chloride and β-chloroisobutyryl chloride are preferred.

Examples of the carbamate or urea compounds of the general formula (III) used according to the invention are methyl carbamate, ethyl carbamate, propyl carbamate, isopropyl carbamate, butyl carbamate, hexyl carbamate, isobutyric carbamate, tert.-butyl carbamate, phenyl carbamate, benzyl carbamate, naphthyl carbamate, methyl urea, ethyl urea, propyl urea, isopropyl urea, butyl urea, isobutyl urea, tert.-butyl urea, phenyl urea and benzyl urea. These starting compounds are for the most part industrially available and may otherwise be prepared, e.g. by methods such as those described in standard organic textbooks (Houben-Weyl, "Methoden der Organischen Chemie", Thieme-Verlag, Stuttgart, 1983, supplementary volume E4).

As already mentioned above, the previously mentioned starting materials are reacted in the presence of a basic acid trap. Examples of suitable amines are trimethylamine, triethylamine, tripropylamine, triisopropylamine, tributylamine, pyridine, 1,8-diazabicyclo[5.4.0.]undec-7-ene and 1,4-diazabicylco[2.2.2]octane. Further examples of basic traps which may be used in the process according to the invention are potassium hydroxide, sodium hydroxide and metallo-organic alkali metal compounds such as sodium methylate and ethylate.

Surprisingly, it was found that the reaction described between a carbamate compound and a urea compound proceeds not only in the presence of the previously mentioned strong bases, but also in the presence of weak bases. Examples of this type of base are salts of weak acids with strong bases such as sodium acetate, potassium acetate, sodium carbonate, potassium carbonate and calcium carbonate. This is especially surprising because in general strong bases such as sodium hydroxide, caustic potash or very high temperatures are required to bond with hydrogen halides from β-chloropropionyl compounds.

Reaction of the β-haloalkanoyl chlorides with carbamates or ureas in the presence of bases may be performed with the starting compounds in a wide range of molar ratios. The following molar ratios are suitable, for instance, base:acid chloride:carbamate ratios of 6:3:2 to 1:1:1, preferably 2:1:1. The reaction temperature is 0 to 100°C, preferably 40 to 80°C, for reactions with carbamates and 0 to 50°C for reactions with the more reactive ureas.

The reaction can generally be performed so that the acid chloride and the carbamate compound or the urea compound are initially introduced. The base is then added continuously during the reaction so that the hydrogen halide formed is trapped.

The reaction can be performed in the melt or in solution. Suitable solvents are those which do not react with the starting compounds such as, for instance, aliphatic and aromatic hydrocarbons, ethers, esters and ketones. Examples of suitable solvents are toluene, petroleum spirit, acetone, 2-butanone, ethyl acetate and dibutyl ether.

In addition, an excess of β-haloalkanoic chloride, base or carbamate can assume the role of a solvent.

To stabilise the products, polymerisation inhibitors such as, for instance, hydroquinone, phenothiazine or butylated hydroxytoluene (BHT), may be added.

In accordance with a modified embodiment of the process, the base is added dropwise only after initiating the reaction. This leads to products with particularly high yields.

The reaction may be performed either continuously or batchwise. The following methods have proved to be useful, wherein either the carbamate compound is initially introduced and the base and acid chloride are added or the carbamate compound and acid chloride are initially introduced and the base is added.

The halide salts, which are produced as solid by-products due to reaction of the hydrogen halide with the base, are removed, for example, by washing out with water or by filtration. After removing the solvent, the colourless, solid acryloyl carbamates or ureas are obtained and these can be purified by crystallisation or distillation under reduced pressure.

Following removal of the halide salts and solvent, the products are generally very pure and do not require further purification.

The acryloyl carbamates and acryloyl ureas prepared by the process according to the invention may be used to prepare addition copolymers which possess the properties of the acylcarbamate and acylurea groups and are suitable as coating materials. These coatings have very good properties with regard to surface adhesion to a variety of substances such as, for instance, glass and metal, as well as particularly high elasticity (EP 0 177 122). The polar structure of the acylcarbamate groups also improves the dispersability of the polymers in aqueous solvent mixtures.

The invention is now explained in more detail by means of the following examples in which the preparation of acryloyl carbamates and ureas is described, starting from carbamate or urea compounds.

### Examples

### Example 1

A mixture of 126 g of β-chloropropionyl chloride, 75 g of methyl carbamate, 200 g of toluene and 2 g of butylated hydroxytoluene (BHT) is heated to 70°C. After the reaction starts, 202 g of triethylamine are added dropwise with stirring in such a way that the hydrogen chloride produced is bonded. After 3 hours the precipitated solid is removed by filtration, extracted with acetone and filtered again. On evaporating down the combined filtrates, the product, N-acryloylmethylcarbamate (H₂C=CH-CO-NH-COO-CH₃), crystallises out as a colourless solid and is isolated by filtration. Yield: 105 g, melting point: 136°C.

### Example 2

A mixture of 126 g of β-chloropropionyl chloride, 89 g of ethyl carbamate, 200 g of toluene and 2 g of butylated hydroxytoluene (BHT) is heated to 70°C. After the reaction starts, 202 g of triethylamine are added dropwise with stirring in such a way that the hydrogen chloride produced is bonded. After 3 hours, the precipitated solid is removed by filtration, extracted with acetone and filtered again. On evaporating down the combined filtrates, the product, N-acryloylethylcarbamate (H₂C=CH-CO-NH-COO-C₂H₅), crystallises out as a colourless solid and is isolated by filtration. Yield: 125 g. The product is purified by recrystallisation from diethyl ether/petroleum ether (1:1). Melting point: 78°C.

### Example 3

A mixture of 126 g of β-chloropropionyl chloride, 103 g of propyl carbamate, 200 g of toluene and 2 g of butylated hydroxytoluene (BHT) is heated to 70°C. After the reaction starts, 202 g of triethylamine are added dropwise with stirring in such a way that the hydrogen chloride being produced is bonded. After 3 hours, the precipitated solid is removed by filtration, extracted with acetone and filtered again. On evaporating down the combined filtrates, the product, N-acryloylpropylcarbamate (H₂C=CH-CO-NH-COO-C₃H₇), crystallises out as a colourless solid and is isolated by filtration. Yield: 154 g; melting point: 69°C.

### Example 4

A mixture of 126 g of β-chloropropionyl chloride, 117 g of butyl carbamate and 3 g of butylated hydroxytoluene (BHT) is heated to 70°C. After the reaction starts, 202 g of triethylamine are added dropwise with stirring in such a way that the hydrogen chloride being produced is bonded. After 3 hours, the precipitated solid is removed by filtration. On evaporating down the filtrate, the product, N-acryloylbutylcarbamate (H₂C=CH-CO-NH-COO-C₄H₉), crystallises out as a colourless solid and is isolated by filtration. Yield: 154 g; melting point: 91°C.

### Example 5

A mixture of 126 g of β-chloropropionyl chloride, 130 g of phenyl carbamate and 3 g of butylated hydroxytoluene (BHT) is heated to 50°C. After the reaction starts, 202 g of triethylamine are added dropwise with stirring in such a way that the hydrogen chloride being produced is bonded. After 3 hours, the precipitated solid is removed by filtration, extracted with acetone and filtered again. On evaporating down the combined filtrates, the product, N-acryloylphenyl-carbamate (H₂C=CH-CO-NH-COO-C₆H₅), crystallises out as a colourless solid and is isolated by filtration. Yield: 160 g; melting point: 134 to 135°C.

### Example 6

A mixture of 126 g of β-chloropropionyl chloride, 74 g of N-methylurea, 200 g of toluene and 2 g of butylated hydroxytoluene (BHT) is stirred at room temperature. After the reaction starts, 202 g of triethylamine is added dropwise with cooling in such a way that the hydrogen chloride being produced is bonded. After 3 hours the precipitated solid is removed by filtration, extracted with acetone and filtered again. On evaporating down the filtrate, the product, N'-acryloyl-N-methylurea (H₂C=CH-CO-NH-CO-NH-CH₃), crystallises out as a colourless solid and is isolated by filtration. Yield: 110 g; melting point: 162°C.

### Example 7

A mixture of 16.4 g of β-chloropropionyl chloride, 7.5 g of methyl carbamate and 0.2 g of butylated hydroxytoluene (BHT) is heated to 80°C. After 2 hours the temperature is lowered to 40°C and 30 g of triethylamine are added dropwise with stirring. After one hour, the precipitated solid is separated by filtration and the filtrate is evaporated down until the colourless product, N-acryloylmethylcarbamate (H₂C=CH-CO-NH-COO-CH₃), precipitates and it is then isolated by filtration. Yield: 10.5 g; melting point: 134°C.

### Example 8

A mixture of 900 g of β-chloropropionyl chloride and 350 g of methyl carbamate are heated at 75°C for 3 hours with stirring. Then a mixture of 3 l of acetone, 720 g of triethylamine and 10 g of butylated hydroxytoluene (BHT) is added. After 3 hours at 40 to 45°C, the precipitated solid is separated by filtration at room temperature. On evaporating down the filtrate, the product, N-acryloylmethylcarbamate (H₂C=CH-CO-NH-COO-CH₃), crystallises out as a colourless solid and is isolated by filtration. Yield: 421 g; melting point: 134 to 136°C.

### Example 9

A mixture of 140 g of β-chloropropionyl chloride and 89 g of ethyl carbamate is heated at 75 to 80°C for 3 hours, with stirring. The excess chloropropionyl chloride is then removed. 50 g of triethylamine are added to 25 g of the residue and stirred for 30 minutes at 80°C. Afterwards, the solid constituents are removed by filtration and the filtrate is evaporated to dryness. Purification is performed by recrystallisation from toluene. Yield: 19 g of N-acryloylethylcarbamate (H₂C=CH-CO-NH-COO-C₂H₅); melting point: 79°C.

### Example 10

The same procedure as in example 9, but pyridine is used instead of triethylamine. Yield: 17 g; melting point: 79°C.

### Example 11

A mixture of 720 g of β-chloropropionyl chloride and 250 g of N-methylurea is stirred for 2 hours at 70°C. After cooling, a mixture of 450 g of triethylamine, 1000 ml of methanol and 3 g of butylated hydroxytoluene is added. After 3 hours the precipitated solid is removed by filtration. On evaporating down the filtrate, the product, N'-acryloyl-N-methylurea (H₂C=C-CO-NH-CO-NH-CH₃), crystallises out as a colourless solid and is isolated by filtration. Yield: 110 g; melting point: 162°C.

### Example 12

A mixture of 500 g of β-chloropropionyl chloride, 300 g of N-butylurea and 300 g of toluene is stirred for 3 hours at 80°C. Then a mixture of 300 g of triethylamine and 2 l of acetone containing 3 g of butylated hydroxytoluene is added and stirred for 3 hours at 40°C. The precipitated solid is removed by filtration. On evaporating down the filtrate, the product, acryloyl-N-butylurea (H₂C=CH-CO-NH-CO-NH-C₄H₉), crystallises out as a colourless solid and is isolated by filtration. Yield: 235 g; melting point: 66°C.

### Example 13

A mixture of 258 g of β-chloropropionyl chloride, 200 g of butyl carbamate and 3 g of butylated hydroxytoluene (BHT) is heated to 80°C. After 2 hours, 200 g of toluene are added and, after cooling, the liquid constituents are removed. 140 g of the residue are dissolved in 500 ml of 2-butanone, 0.6 g of butylated hydroxytoluene are added and 90 g of triethylamine are added dropwise at 40°C. After 2 hours, the precipitated solid is removed by filtration and the filtrate is evaporated to dryness. Yield: 114 g of N-acryloylbutylcarbamate (H₂C=CH-CO-NH-CO-O-C₄H₉); melting point: 90°C.

### Example 14

A mixture of 25 g of phenyl carbamate, 35 g of β-chloropropionyl chloride, 0.5 g of butylated hydroxytoluene (BHT) and 100 ml of toluene are stirred for 3 hours at 70°C. Then a mixture of 50 g of triethylamine and 200 ml of acetone is added. After 2 hours at 40 to 45°C, the precipitated salt is removed by filtration and the solvent acetone and excess triethylamine are removed by distillation. A colourless product, N-acryloylphenylcarbamate (H₂C=CH-CO-NH-CO-O-C₆H₅), remains. Yield: 20 g; melting point: 135°C.

### Example 15

A mixture of 28 g of benzylcarbamate, 35 g of β-chloropropionyl chloride, 0.5 g of butylated hydroxytoluene (BHT) and 100 ml of toluene are stirred for 3 hours at 70°C. Then a mixture of 80 g of triethylamine and 200ml of acetone is added. After 2 hours at 40 to 45°C, the precipitated salt is removed by filtration and the solvent acetone and excess triethylamine are removed by distillation. The colourless product N-acryloylbenzylcarbamate (H₂C=CH-CO-NH-CO-O-CH₂-C₆H₅) remains. Yield: 20 g; melting point: 98°C.

### Example 16

A mixture of 25 g of phenylurea, 35 g of β-chloropropionyl chloride, 0.5 g of butylated hydroxytoluene (BHT) and 100 ml of toluene are stirred for 2 hours at 60°C. Then a mixture of 50 g of triethylamine and 200 ml of acetone is added. After 2 hours at 40 to 45°C, the precipitated salt is removed by filtration and the solvent acetone and excess triethylamine are removed by distillation. The colourless product acryloyl-N-phenylurea (H₂C=CH-CO-NH-CO-NH-C₆H₅) remains. Yield: 21 g; melting point: 147°C.

### Example 17

A mixture of 20.4 g of β-chloroisobutyryl chloride, 7.5 g of methyl carbamate and 0.2 g of butylated hydroxytoluene (BHT) is heated to 80°C. After 2 hours the temperature is lowered to 40°C and 35 g of triethylamine and 100 ml of toluene are added dropwise with stirring. After one hour the precipitated solid is removed by filtration and the filtrate is evaporated down until the colourless product N-methacryloylmethyl-carbamate (H₂C=C(CH₃)-CO-NH-COO-CH₃) precipitates, this then being isolated by filtration. Yield: 10.1 g; melting point: 94°C.

### Example 18

A mixture of 20.4 g of β-bromopropionyl chloride, 7.5 g of methyl carbamate, 30 g of triethylamine, 100 g of methylglycol acetate and 0.2 g of butylated hydroxytoluene (BHT) is heated to 80°C. After 2 hours, the precipitated solid is removed by filtration and the filtrate is evaporated down until the colourless product N-acryloylmethylcarbamate (H₂C=CH-CO-NH-COO-CH₃) precipitates, this then being isolated by filtration. Yield: 9.5 g; melting point: 136°C.

### Example 19

A mixture of 18.4 g of β-chloropropionyl chloride, 7.5 g of methyl carbamate, 20 g of sodium acetate, 100 g of methylglycol acetate and 0.2 g of butylated hydroxytoluene (BHT) is heated to 100°C. After 2 hours, the precipitated solid is removed by filtration and the filtrate is evaporated down until the colourless product N-acryloylmethylcarbamate (H₂C=CH-CO-NH-COO-CH₃) precipitates, this then being isolated by filtration. Yield: 9.5 g; melting point: 136°C.

### Example 20

A mixture of 20 g of β-chloropropionyl chloride, 7.5 g of methylurea, 20 g of sodium acetate, 100 g of methylglycol acetate and 0.2 g of butylated hydroxytoluene (BHT) is heated to 100°C. After 2 hours the precipitated solid is removed by filtration and the filtrate evaporated down until the colourless product N-acryloylmethylurea (H₂C=CH-CO-NH-CO-NH-CH₃) precipitates, this then being isolated by filtration. Yield: 9.9 g; melting point: 163°C.

## Claims

1. A process for preparing an acryloyl carbamate or acryloyl urea of the general formula (I) 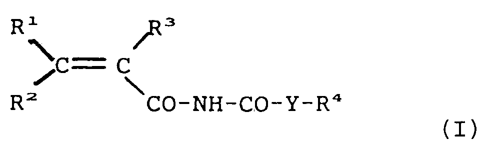 in which R^{1,} R² and R³ may be identical or different and each represents a hydrogen atom or a methyl or ethyl group, R⁴ represents a straight-chain or branched, saturated or unsaturated aliphatic, cycloaliphatic, arylaliphatic or aromatic hydrocarbon group with 1 to 10 carbon atoms, Y represents an oxygen atom or the NR⁵ group, wherein R⁵ represents a hydrogen atom or a straight-chain or branched, saturated or unsaturated aliphatic, cycloaliphatic, arylaliphatic or aromatic hydrocarbon group with 1 to 6 carbon atoms,
characterised in that a β-haloalkanoyl chloride of the general formula (II) 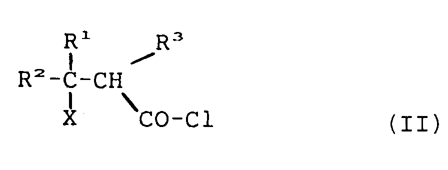 in which R¹, R² and R³ are defined as above and X represents a chlorine or bromine atom
is reacted with a carbamate or urea compound of the general formula (III)
H₂N-CO-Y-R⁴ (III)
in which R⁴ and Y are defined as above, at temperatures of 0 to 100°C and in the presence of a trap for the hydrogen halide produced during reaction.

2. A process according to Claim 1, characterised in that the trap is selected from the group comprising tertiary amines and alkali or alkaline earth metal oxides, hydroxides, carbonates, alkoxides and carboxylates.

3. A process according to Claim 1 or 2, characterised in that the trap for the hydrogen halide produced during reaction is added dropwise after initiating the reaction.

4. A process according to one of Claims 1 to 3, characterised in that the trap for the hydrogen halide produced during reaction is one or more compounds from the group of acetates and carbonates.

5. A process according to Claim 4, characterised in that the trap for the hydrogen halide produced during reaction is sodium acetate, potassium acetate, sodium carbonate, potassium carbonate, sodium hydrogen carbonate, potassium hydrogen carbonate and/or calcium carbonate.

6. A process according to one of Claims 1 to 5, characterised in that in the case of reactions involving carbamate compounds the reaction is performed at temperatures of 40 to 80°C.

7. A process according to one of Claims 1 to 5, characterised in that in the case of reactions involving urea compounds the reaction is performed at temperatures of 0 to 50°C.
